# EUROPEAN PATENT APPLICATION

(11) **EP 1 398 314 A1**
(43) Date of publication of application: **17.03.2004**
(21) Application number: 02771714.9
(22) Date of filing: 17.05.2002
(51) Int. Cl.: C07D 477/20, A61K 31/407, A61P 31/04

(54) **CARBAPENEM COMPOUND**

(30) Priority: 21.05.2001 JP 2001150874
(71) Applicant: Kyoto Pharmaceutical Industries, Ltd., Kyoto-shi, Kyoto 604-8444 (JP)
(72) Inventor: MATSUI, Hiroshi, Nara-shi, Nara 631-0074 (JP)
(74) Representative: von Kreisler, Alek, Dipl.-Chem.
(86) International application number: PCT/JP2002/004767
(87) International publication number: WO 2002/094829

(57) **Abstract**

A carbapenem compound represented by the formula (I'): 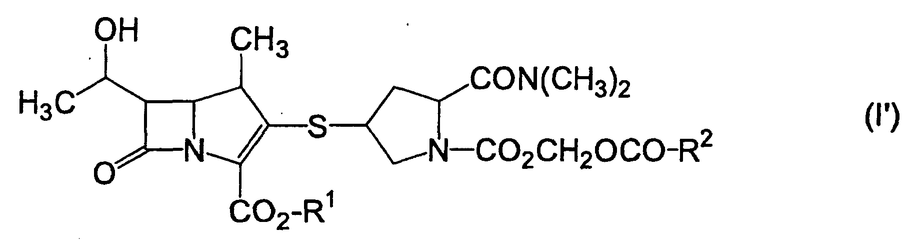 wherein each symbol is as defined in the specification, and an antibacterial agent containing this compound as an active ingredient are provided.

The carbapenem compound of the present invention is superior in absorbability from the gastrointestinal tract by oral administration and extremely useful for the prophylaxis or treatment of infectious diseases (particularly, bacterial infectious diseases).

## Description

### Technical Field

The present invention relates to a novel carbapenem compound useful as a prophylactic or therapeutic agent for bacterial infectious diseases and the like. More particularly, the present invention relates to an antibacterial agent containing this compound as an active ingredient, which has superior oral absorbability and which shows sufficient antibacterial property.

### Background Art

There have been found many compounds having a carbapenem skeleton, as therapeutic agents for infectious diseases, from which several carbapenem compounds having superior antibacterial activity have been put to practical use, or under development for practicalization. For example, a carbapenem compound (meropenem) represented by the formula (A) 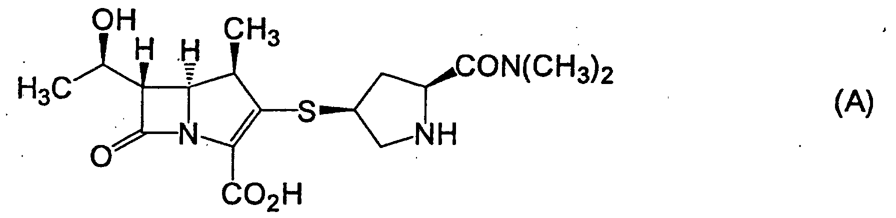 has been already put to practical use and clinically applied. Meropenem has a broad antibacterial spectrum and strong antibacterial activity, and has overcome instability to renal dehydropeptidase, which has been conventionally considered to be the defect of carbapenem compounds. It has superior characteristics in that it can be administered alone without using a stabilizer in combination.

However, meropenem shows poor absorbatility from the gastrointestinal tract, which only permits clinical administration in the form of injection. Oral agents are easy and convenient to administer as compared to injection, and clinically extremely highly useful. Thus, there is a strong demand for the development of a carbapenem compound for oral administration, which has strong antibacterial activity and broad antibacterial spectrum, as well as superior gastrointestinal tract absorbability.

### Disclosure of the Invention

It is an object of the present invention to provide a useful compound having superior absorbability from the gastrointestinal tract, and strong antibacterial property upon hydrolysis after absorption.

The present inventor has conducted intensive studies with the aim of achieving the above-mentioned objects and found that a specific carbapenem compound (prodrug) represented by the following formula (I') shows superior absorbability from the gastrointestinal tract as compared to conventionally proposed carbapenem compounds for oral administration, is deesterified in the body into a carbapenem compound having antibacterial property and then exerts antibacterial property, which resulted in the completion of the present invention.

Accordingly, the present invention provides the following.
(1) A carbapenem compound represented by the formula (I'): 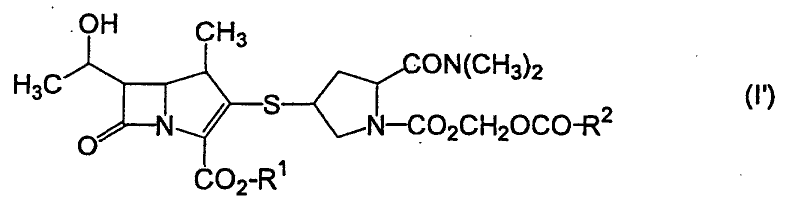 wherein
   - R¹: is -CH₂OCOC(CH₃)₃, -CH₂OCO₂CH(C₂H₅)₂, -CH(CH₃)OCO₂CH(C₂H₅)₂, 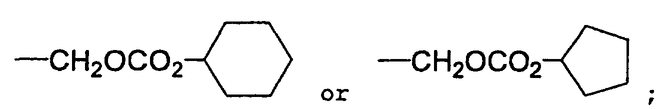 and
   - R²: is -CH₂CH₂CH₃ or -CH₂CH₃, -CH(CH₃)₂,
   provided that (i) when R¹ is -CH₂OCOC(CH₃)₃, then R² should be -CH₂CH₂CH₃,
   (ii) when R¹ is -CH₂OCO₂CH(C₂H₅)₂ or -CH(CH₃)OCO₂CH(C₂H₅)₂, then R² should be -CH₂CH₂CH₃, -CH₂CH₃ or -CH(CH₃)₂, and
   (iii) when R¹ is 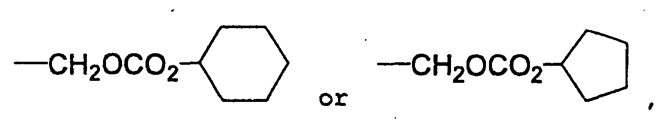 then R² should be -CH₂CH₂CH₃ or -CH(CH₃)₂.
(2) The compound of the above-mentioned (1), which is pivaloyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
   1-ethylpropyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
   1-ethylpropyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
   1-ethylpropyloxycarbonyloxymethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate,
   1-(1-ethylpropyloxycarbonyloxy)ethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
   1-(1-ethylpropyloxycarbonyloxy)ethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
   1-(1-ethylpropyloxycarbonyloxy)ethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate,
   cyclohexyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
   cyclohexyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
   cyclopentyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate or
   cyclopentyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate.
(3) An antibacterial agent containing the compound of the above-mentioned (1) or (2) as an active ingredient.
(4) The antibacterial agent of the above-mentioned (3), which is used for oral administration.
(5) A carbapenem compound represented by the formula (I): 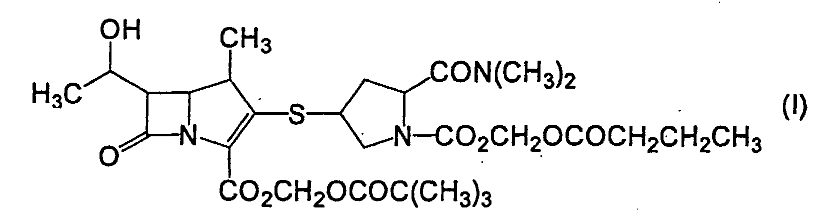
(6) The compound of the above-mentioned (5), which is pivaloyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate.
(7) An antibacterial agent containing the compound of the above-mentioned (5) or (6) as an active ingredient.
(8) The antibacterial agent of the above-mentioned (7), which is used for oral administration.

### Detailed Description of the Invention

The present invention is explained in detail in the following.

The carbapenem compound of the present invention, which is represented by the following formula (I'): 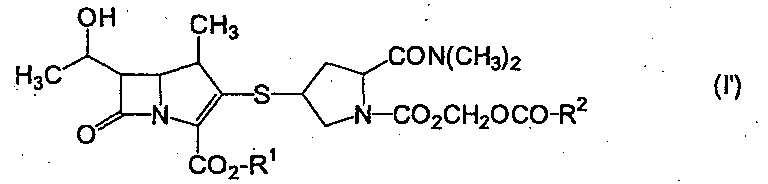 wherein each symbol is as defined above, such as the formula (I) : 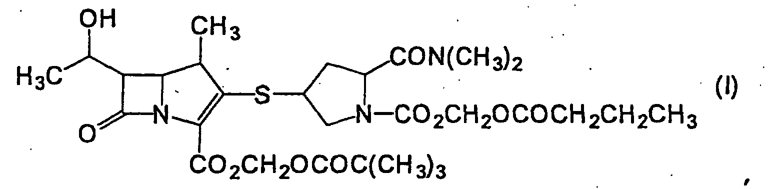 is superior in absorbability from the gastrointestinal tract as compared to conventional carbapenem compounds for oral administration. The antibacterial agent of the present invention contains compound (I') as an active ingredient.

The compound (I'), such as compound (I), can be produced by the following production method or a method analogous thereto. Other compounds encompassed in compound (I') can be also produced according to a method analogous to the production method of the following compound (I). 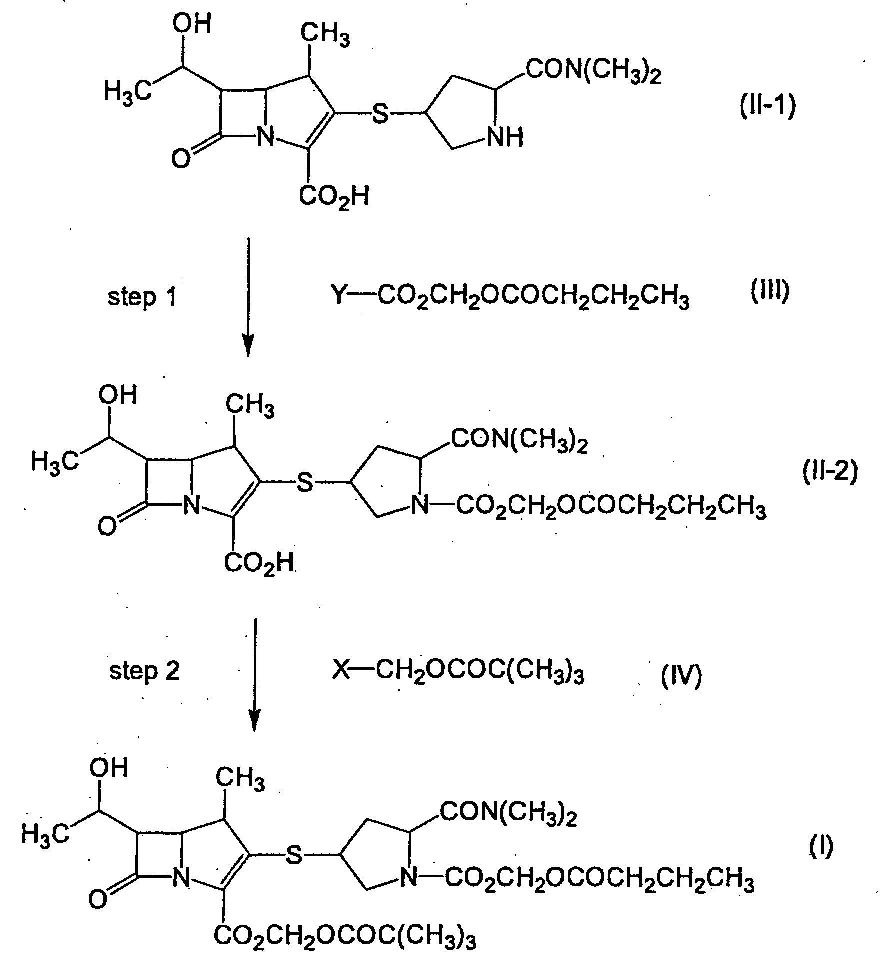 wherein X is a leaving group such as a halogen atom (e.g., chlorine atom, bromine atom or iodine atom and the like), an alkanesulfonyloxy group (e.g., methanesulfonyloxy, ethanesulfonyloxy, propanesulfonyloxy, butanesulfonyloxy and the like), an arylsulfonyloxy group (e.g., phenylsulfonyloxy, tolylsulfonyloxy and the like), and the like, and Y is a leaving group such as a chlorine atom, imidazol-1-yl, p-nitrophenyloxy, 2-phenylacetonitrile-2-yl-iminooxy group and the like.

### Step 1

The compound (II-2) can be obtained by reacting compound (II-1) or a salt thereof with compound (III) in a solvent that does not inhibit the reaction, such as dioxane, acetonitrile, tetrahydrofuran, chloroform, methylene chloride, ethylene chloride, benzene, ethyl acetate, N,N-dimethylformamide, N,N-dimethylacetamide, dimethyl sulfoxide and the like, or a mixture thereof. The amount of compound (III) to be used is generally about 1-5 moles, preferably about 1-2 moles, relative to one mole of compound (II-1). The compound (II-1) can be obtained by a method described in, for example, JP-B-63-55514 and the like.

As the salt of compound (II-1), for example, alkali metal salts such as sodium salt, potassium salt and the like, alkaline earth metal salts such as calcium salt and the like, triethylamine salt, dicyclohexylamine salt, pyridine salt and the like can be mentioned.

The reaction of Step 1 can be also carried out in the presence of a base. The base to be used is subject to no particular limitation but preferably, inorganic bases such as sodium hydrogencarbonate, potassium carbonate and the like and organic bases such as triethylamine, diisopropylethylamine, pyridine and the like can be mentioned.

The reaction temperature in Step 1 is not particularly limited but this step is preferably performed at comparatively low temperature to suppress side reactions, which is generally -30°C to 40°C, preferably -10°C to 10°C. While the reaction time varies depending mainly on reaction temperature, the kind of reaction reagent and the like, it is generally 30 min to dozen hours or so.

### Step 2

The compound (I) can be obtained by reacting compound (II-2) or a salt thereof with compound (IV) in a solvent that does not inhibit the reaction, such as dioxane, acetonitrile, tetrahydrofuran, chloroform, methylene chloride, ethylene chloride, benzene, ethyl acetate, N,N-dimethylformamide, N,N-dimethylacetamide, dimethyl sulfoxide and the like, or a mixture thereof preferably in the presence of a base. The amount of compound (IV) to be used is generally about 1-5 moles, preferably about 1-2 moles, relative to one mole of compound (II-2).

When compound (II-2) is not in the form of a salt, the reaction of Step 2 is carried out in the presence of a base. The base to be used is subject to no particular limitation but preferably, inorganic bases such as sodium hydrogencarbonate, potassium carbonate and the like and organic bases such as triethylamine, diisopropylethylamine, pyridine and the like can be mentioned. The amount of the base to be used is generally about 1-5 moles, preferably about 1-2 moles, relative to one mole of compound (II-2).

While the reaction temperature of Step 2 is not particularly limited, this step is preferably performed at a comparatively low temperature to suppress side reactions, which is generally -30°C to 40°C, preferably -10°C to 10°C. While the reaction time varies depending mainly on reaction temperature, the kind of reaction reagent and the like, it is generally 30 min to dozen hours or so.

As the salt of compound (II-2), for example, alkali metal salts such as sodium salt, potassium salt and the like, alkaline earth metal salts such as calcium salt and the like, triethylamine salt, dicyclohexylamine salt, pyridine salt and the like can be mentioned.

Where necessary, The compound (I'), such as (I), can be purified by a conventional method, such as recrystallization, preparative thin-layer chromatography, column chromatography and the like.

The compound (I') having a preferable configuration is a compound represented by the following formula (I'-a): 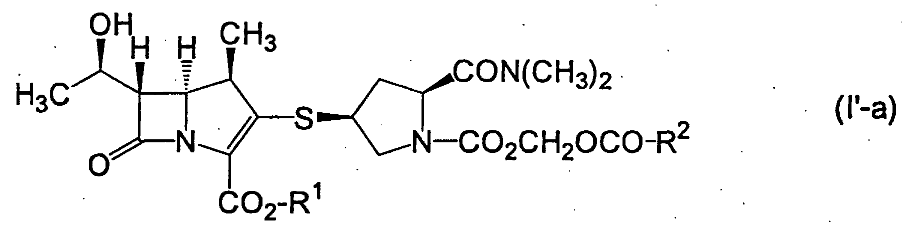 wherein each symbol is as defined above.

The compound (I) having a preferable configuration is the following compound represented by the formula (I-a): 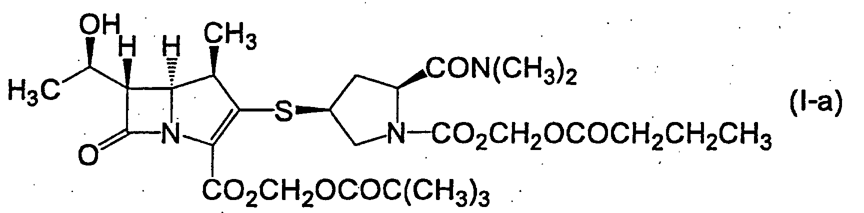 namely, pivaloyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate.

The compound (I') is quickly absorbed into blood by oral administration, becomes the above-mentioned carbapenem compound represented by the formula (II-1) as a metabolite thereof, and shows high blood concentration. Namely, compound (I') is superior in absorbability from the gastrointestinal tract, and is useful as a prodrug of carbapenem compound (particularly meropenem).

Accordingly, a prophylactic or therapeutic agent for infectious diseases, which contains compound (I'), shows the aforementioned superior action by oral administration, and is generally administered as an oral agent.

This prophylactic or therapeutic agent for infectious diseases can be produced by diluting the compound with pharmaceutical excipients according to a method known *per se.* As the excipient, for example, starch, lactose, sugar, calcium carbonate, calcium phosphate and the like can be used.

The prophylactic or therapeutic agent for infectious diseases may contain other additives on demand, and, for example, a binder (e.g., starch, gum arabic, carboxymethyl cellulose, hydroxypropyl cellulose, crystalline cellulose etc.), a lubricant (e.g., magnesium stearate, talc etc.), a disintegrant (e.g., carboxymethyl cellulose calcium, talc etc.) and the like can be mentioned as preferable additives. After mixing various ingredients, the mixture is formulated into a dosage form suitable for oral administration, such as capsule, tablet, fine granule, granule, dry syrup and the like, according to a method known *per se*, whereby an oral prophylactic or therapeutic agent for infectious diseases can be produced.

The compound (I') of the present invention is useful for the prophylaxis and/or treatment of infectious diseases (particularly, bacterial infectious disease) in mammals (human, cow, horse, dog, cat, rat, mouse, hamster and the like). As the infectious diseases, purulent disease, respiratory infectious disease, biliary tract infectious disease, urinary tract infectious disease and the like can be mentioned.

While the dose of compound (I'), particularly (I), varies depending on the administration subject, symptom, and other factors, when it is administered for, for example, adult purulent disease, it is orally administered in a dose of, for example, about 1-40 mg/kg body weight (preferably a dose of about 1-10 mg/kg body weight) about 1 to 4 times a day.

The compound (I') of the present invention can exhibit a superior antibacterial action in a body at a dose smaller than the conventionally proposed carbapenem compound for oral administration.

In addition, compound (I') may be used along with a different antibacterial active substance, such as antibacterial agents (penicillins, aminoglycosides, cephalosporins etc.) or a therapeutic drug for systemic symptoms of bacterial infection (antipyretic, analgesic, antiphlogistic etc.).

### Examples

The physical property and production methods of the compound of the present invention are explained in detail by referring Examples, which are not to be construed as limitative.

### Example 1

### pivaloyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (21.1 g) was suspended in N,N-dimethylformamide (80 ml) and butyryloxymethyl p-nitrophenyl carbonate (16.3 g) was added at 5°C. The mixture was stirred at room temperature for 1 hr.

Again, the reaction solution was cooled to 5°C, and pivaloyloxymethyl iodide (21.4 g) was added. The mixture was stirred at room temperature for 1 hr and ethyl acetate (400 ml) was added. The mixture was washed with 5% brine (400 ml) and dried over anhydrous sodium sulfate.

Ethyl acetate was evaporated under reduced pressure and the residue was purified by silica gel column chromatography. The obtained solid was dissolved in ethyl acetate (40 ml). Isopropyl ether (60 ml) was added and the mixture was stirred for 1 hr. The precipitated crystals were collected by filtration to give 19.7 g of the title compound.
IR (Nujol, cm⁻¹): 3395, 1794, 1753, 1728, 1636
¹H-NMR (CDCl₃, δppm): 0.94 (3H, t), 1.22 (9H, s), 1.26 (3H, d), 1.33 (3H, d) , 1.65 (2H, m) , 2.34 (2H, t) , 1.7-2.8 (3H, m) , 2.9-3.2 (6H), 3.1-3.9 (4H, m), 3.9-4.4 (3H, m), 4.71 (1H, m), 5.6-5.8 (2H, m), 5.89 (2H, ABq).

### Example 2

### 1-ethylpropyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and isobutyryloxymethyl p-nitrophenyl carbonate (813 mg) was added at 5°C. The mixture was stirred at room temperature for 1 hr.

Again, the reaction solution was cooled to 5°C, and 1-ethylpropyloxycarbonyloxymethyl iodide (1.42 g) was added. The mixture was stirred at room temperature for 1 hr and ethyl acetate (50 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (2.5 ml). Isopropyl ether (5.5 ml) was added and the mixture was stirred for 1 hr. The precipitated crystals were collected by filtration to give 1.22 g of the title compound.
IR (Nujol, cm⁻¹): 3395, 1759, 1724, 1651
¹H-NMR (CDCl₃, δppm): 0.91 (6H, t), 1.17 (6H, d), 1.25 (3H, d), 1.33 (3H, d), 2.9-3.2 (6H), 3.1-3.9 (4H, m), 3.9-4.4 (3H, m), 4.5-4.8 (2H, m), 5.6-6.0 (4H, m).

### Example 3

### 1-ethylpropyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio)-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (5 ml) and butyryloxymethyl p-nitrophenyl carbonate (813 mg) was added at 5°C. The mixture was stirred at room temperature for 1 hr.

Again, the reaction solution was cooled to 5°C, and 1-ethylpropyloxycarbonyloxymethyl iodide (1.47 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the mixture was stirred for 1 hr. The precipitated crystals were collected by filtration to give 1.02 g of the title compound.
IR (Nujol, cm⁻¹): 3400, 1761, 1722, 1651
¹H-NMR (CDCl₃, δppm): 0.92 (6H, t), 0.94 (3H, t), 1.26 (3H, d), 1.33 (3H, d), 1.63 (4H, qui), 2.34 (2H, t), 2.9-3.2 (6H), 3.1-3.9 (4H, m), 3.9-4.4 (3H, m), 4.5-4.8 (2H, m), 5.6-6.0 (4H, m).

### Example 4

### 1-ethylpropyloxycarbonyloxymethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and propionyloxymethyl p-nitrophenyl carbonate (773 mg) was added at 5°C. The mixture was stirred at room temperature for 1 hr.

Again, the reaction solution was cooled to 5°C, and 1-ethylpropyloxycarbonyloxymethyl iodide (1.42 g) was added. The mixture was stirred at room temperature for 1 hr and ethyl acetate (100 ml) was added. The mixture was washed with 5% brine (100 ml) and dried over anhydrous sodium sulfate.

Ethyl acetate was evaporated under reduced pressure and the residue was purified by silica gel column chromatography. The obtained solid was dissolved in ethyl acetate (4 ml). Isopropyl ether (20 ml) was added and the precipitated crystals were collected by filtration to give 1.13 g of the title compound.
IR (Nujol, cm⁻¹): 3395, 1761, 1717, 1653
¹H-NMR (CDCl₃, δppm): 0.92 (6H, t), 1.14 (3H, t), 1.26 (3H, d), 1.33 (3H, d), 1.64 (4H, qui), 2.39 (2H, q), 2.9-3.2 (6H), 3.1-3.9 (4H, m), 3.9-4.4 (3H, m), 4.6-4.8 (2H, m), 5.6-6.0 (4H, m).

### Example 5

### 1-(1-ethylpropyloxycarbonyloxy)ethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and isobutyryloxymethyl p-nitrophenyl carbonate (813 mg) was added at 5°C. The mixture was stirred at room temperature for 1 hr.

Again, the reaction solution was cooled to 5°C, and 1-(1-ethylpropyloxycarbonyloxy)ethyl iodide (1.49 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 810 mg of the title compound.
IR (Nujol, cm⁻¹): 3395, 1755, 1728, 1651
¹H-NMR (CDCl₃, δppm): 0.91 (6H, t), 1.18 (6H, d), 1.26 (3H, d), 1.33 (3H, d), 1.5-1.7 (4H, m), 1.58, 1.59 (3H, d), 2.9-3.2 (6H), 3.1-3.9 (4H, m), 3.9-4.4 (3H, m), 4.5-4.8 (2H, m), 5.6-5.8 (2H, m), 6.86 (1H, q).

### Example 6

### 1-(1-ethylpropyloxycarbonyloxy)ethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and butyryloxymethyl p-nitrophenyl carbonate (813 mg) was added at 5°C. The mixture was stirred at room temperature for 1 hr.

Again, the reaction solution was cooled to 5°C, and 1-(1-ethylpropyloxycarbonyloxy)ethyl iodide (1.49 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 650 mg of the title compound.
¹H-NMR (CDCl₃, δppm): 0.91, 0.92 (6H, t), 0.94 (3H, t), 1.25 (3H, d), 1.33 (3H, d), 1.58 (3H, d), 2.34(2H, t), 2.9-3.2 (6H), 3.1-3.9 (4H, m), 3.9-4.4 (3H, m), 4.5-4.8 (2H, m), 5.6-5.8 (2H, m), 6.86 (1H, q).

### Example 7

### 1-(1-ethylpropyloxycarbonyloxy)ethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and propionyloxymethyl p-nitrophenyl carbonate (773 mg) was added at 5°C. The mixture was stirred at room temperature for 1 hr.

Again, the reaction solution was cooled to 5°C, and 1-(1-ethylpropyloxycarbonyloxy)ethyl iodide (1.49 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 790 mg of the title compound.
IR (Nujol, cm⁻¹): 3395, 1759, 1728, 1651
¹H-NMR (CDCl₃, δppm): 0.91 (6H, t), 1.14 (3H, d), 1.26 (3H, d), 1.33 (3H, d), 1.59, 1.60 (3H, d), 2.39 (2H, q), 2.9-3.2 (6H), 3.1-3.9 (4H, m), 3.9-4.4 (3H, m), 4.5-4.8 (2H, m), 5.6-5.8 (2H, m), 6. 86 (1H, q).

### Example 8

### cyclohexyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and isobutyryloxymethyl p-nitrophenyl carbonate (813 mg) was added at 5°C. The mixture was stirred at room temperature for 1 hr.

Again, the reaction solution was cooled to 5°C, and cyclohexyloxycarbonyloxymethyl iodide (1.48 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 1.19 g of the title compound.
IR (Nujol, cm⁻¹): 3395, 1796, 1751, 1728, 1638
¹H-NMR (CDCl₃, δppm): 1.18 (6H, d), 1.0-2.1 (11H, m), 1.26 (3H, d), 1.33 (3H, d), 2.4-2.9 (2H, m), 2.9-3.2 (6H), 3.1-3.9 (4H, m), 3.9-4.4 (3H, m), 4.6-4.8 (2H, m), 5.6-6.0 (4H, m).

### Example 9

### cyclohexyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and butyryloxymethyl p-nitrophenyl carbonate (813 mg) was added at 5°C. The mixture was stirred at room temperature for 1 hr.

Again, the reaction solution was cooled to 5°C, and cyclohexyloxycarbonyloxymethyl iodide (1.64 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 1.10 g of the title compound.
IR (Nujol, cm⁻¹): 3400, 1760, 1715, 1653
¹H-NMR (CDCl₃, δppm): 0.95 (3H, t), 1.1-2.2 (13H, m), 1.30 (3H, d), 1.34 (3H, d), 2.34 (2H, t), 2.5-2.9 (1H, m), 2.9-3.2 (6H), 3.1-3.9 (4H, m), 3.9-4.4 (3H, m), 4.5-4.9 (2H, m), 5.6-5.9 (2H, m), 5.84, 5.92 (2H, ABq).

### Example 10

### cyclopentyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and isobutyryloxymethyl p-nitrophenyl carbonate (813 mg) was added at 5°C. The mixture was stirred at room temperature for 1 hr.

Again, the reaction solution was cooled to 5°C, and cyclopentyloxycarbonyloxymethyl iodide (1.45 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 1.01 g of the title compound.
¹H-NMR (CDCl₃, δppm): 1.18 (6H, d), 1.0-2.1 (9H, m), 1.26 (3H, d), 1.33 (3H, d), 2.4-2.9 (2H, m), 2.9-3.2 (6H), 3.1-3.9 (4H, m), 3.9-4.4 (3H, m), 4.6-4.8 (2H, m), 5.6-6.0 (4H, m).

### Example 11

### cyclopentyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate

Sodium (1R,5S,6S)-2-[(3S,5S)-5-N,N-dimethylaminocarbonylpyrrolidin-3-ylthio]-6-[(1R)-1-hydroxyethyl]-1-methylcarbapen-2-em-3-carboxylate (1.06 g) was suspended in N,N-dimethylformamide (4 ml) and butyryloxymethyl p-nitrophenyl carbonate (813 mg) was added at 5°C. The mixture was stirred at room temperature for 1 hr.

Again, the reaction solution was cooled to 5°C, and cyclopentyloxycarbonyloxymethyl iodide (1.45 g) was added. The mixture was stirred at room temperature for 1 hr and ethyl acetate (100 ml) was added. The mixture was washed with 5% brine (50 ml) and dried over anhydrous sodium sulfate.

Ethyl acetate was evaporated under reduced pressure and the residue was purified by silica gel column chromatography. The obtained solid was dissolved in ethyl acetate (5 ml). Isopropyl ether (20 ml) was added and the precipitated powder was collected by filtration to give 0.95 g of the title compound.
¹H-NMR (CDCl₃, δppm): 0.95 (3H, t), 1.1-2.2 (11H, m), 1.30 (3H, d), 1.34 (3H, d), 2.34 (2H, t), 2.5-2.9 (1H, m), 2.9-3.2 (6H), 3.1-3.9 (4H, m), 3.9-4.4 (3H, m), 4.5-4.9 (2H, m), 5.6-6.0 (4H, m).

The chemical structural formulas of the compounds obtained in Examples 1-11 are shown in Table 1.

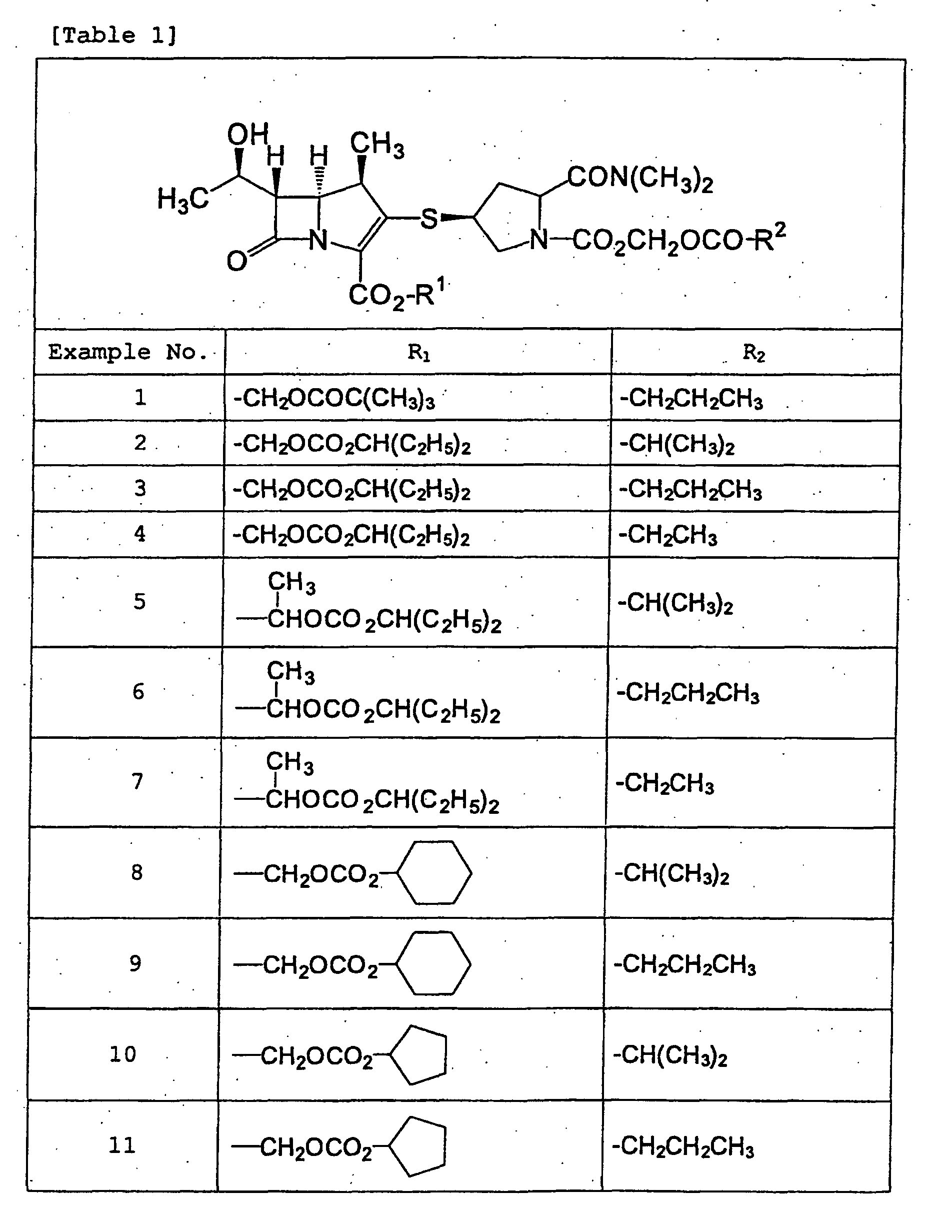

### Experimental Example

To clarify the superior properties of the compounds of the present invention, the following oral absorption test was performed.

### Experimental Example 1

An enzyme inhibitor (cilastatin) (50 mg/kg) was pre-administered to rats (3 per group) and the compound of the present invention (compound of Example 1, 20 mg/kg) was orally administered. The plasma concentration of meropenem produced by hydrolysis was measure at 0.125, 0.25, 0.5, 1.0, 1.5, 2.0 and 3.0 hr by HPLC and the area under plasma concentration - time curve (AUC) was determined. The results are shown in Table 2.

**[Table 2]**

| Test compound | Average plasma concentration (µg/ml) | | | | | | | AUC₀₋₃ₕᵣ (µg hr/ml) |
|---|---|---|---|---|---|---|---|---|
| | 0.125 hr | 0.25 hr | 0.5 hr | 1.0 hr | 1.5 hr | 2.0 hr | 3.0 hr | |
| Example 1 | 0.62 | 1.31 | 2.31 | 2.59 | 2.27 | 1.56 | 0.55 | 5.06 |

### Industrial Applicability

The compound (I') of the present invention is superior in absorbability from the gastrointestinal tract by oral administration as compared to conventionally proposed carbapenem compounds for oral administration, and the active form produced in living organism shows sufficient antibacterial property to a wide range of bacterial strains. Therefore, it is extremely useful for the prophylaxis or treatment of infectious diseases (particularly, bacterial infectious diseases).

This application is based on a patent application No. 2001-150874 filed in Japan, the contents of which are hereby incorporated by reference.

## Claims

1. A carbapenem compound represented by the formula (I'): 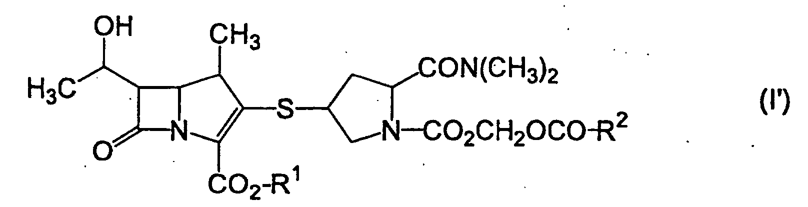 wherein
R¹ is -CH₂OCOC(CH₃)₃, -CH₂OCO₂CH(C₂H₅)₂, -CH(CH₃)OCO₂CH(C₂H₅)₂, 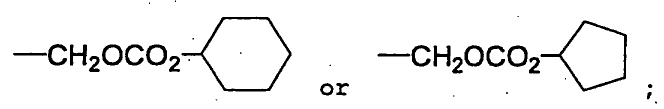 and
R² is -CH₂CH₂CH₃ or -CH₂CH₃, -CH(CH₃)₂,
provided that (i) when R¹ is -CH₂OCOC(CH₃)₃, then R² should be -CH₂CH₂CH₃,
(ii) when R¹ is -CH₂OCO₂CH(C₂H₅)₂ or -CH(CH₃)OCO₂CH(C₂H₅)₂, then R² should be -CH₂CH₂CH₃, -CH₂CH₃ or -CH(CH₃)₂, and
(iii) when R¹ is 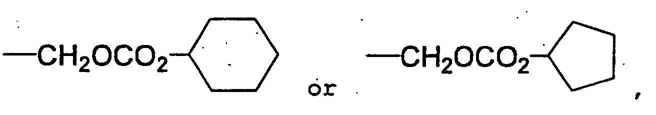 then R² should be -CH₂CH₂CH₃ or -CH(CH₃)₂.

2. The compound of claim 1, which is pivaloyloxymethyl (1R,5S,6S)-2-[(3S,5S)-(5-N,N-dimethylaminocarbonyl-1-butyryloxymethyloxycarbonyl)pyrrolidin-3-ylthio]-6-[(1R)-1-hydroxyethyl]-1-methylcarbapen-2-em-3-carboxylate, 1-ethylpropyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
1-ethylpropyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
1-ethylpropyloxycarbonyloxymethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate,
1-(1-ethylpropyloxycarbonyloxy)ethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
1-(1-ethylpropyloxycarbonyloxy)ethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
1-(1-ethylpropyloxycarbonyloxy)ethyl (1R,5S,6S)-2-[(3S,5S)-(5-N,N-dimethylaminocarbonyl-1-propionyloxymethyloxycarbonyl)-pyrrolidin-3-ylthio]-6-[(1R)-1-hydroxyethyl]-1-methylcarbapen-2-em-3-carboxylate,
cyclohexyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate,
cyclohexyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate,
cyclopentyloxycarbonyloxymethyl (1R,5S,6S)-2-[(3S,5S)-(5-N,N-dimethylaminocarbonyl-1-isobutyryloxymethyloxycarbonyl)-pyrrolidin-3-ylthio]-6-[(1R)-1-hydroxyethyl]-1-methylcarbapen-2-em-3-carboxylate or
cyclopentyloxycarbonyloxymethyl (1R,5S,6S)-2-[(3S,5S)-(5-N,N-dimethylaminocarbonyl-1-butyryloxymethyloxycarbonyl)-pyrrolidin-3-ylthio]-6-[(1R)-1-hydroxyethyl]-1-methylcarbapen-2-em-3-carboxylate.

3. An antibacterial agent comprising the compound of claim 1 or 2 as an active ingredient.

4. The antibacterial agent of claim 3, which is used for oral administration.

5. A carbapenem compound represented by the formula (I):

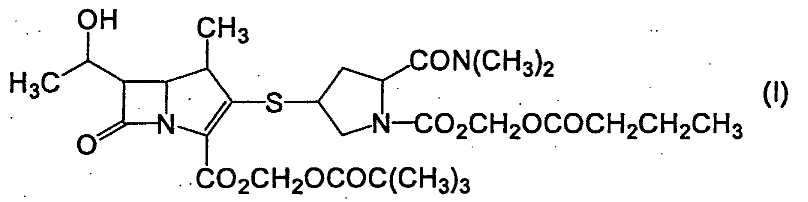

6. The compound of claim 5, which is pivaloyloxymethyl (1R,5S,6S)-2-[(3S,5S)-(5-N,N-dimethylaminocarbonyl-1-butyryloxymethyloxycarbonyl)pyrrolidin-3-ylthio]-6-[(1R)-1-hydroxyethyl]-1-methylcarbapen-2-em-3-carboxylate.

7. An antibacterial agent comprising the compound of claim 5 or 6 as an active ingredient.

8. The antibacterial agent of claim 7, which is used for oral administration.
